# EUROPEAN PATENT APPLICATION

(11) **EP 4 265 269 A1**
(43) Date of publication of application: **25.10.2023**
(21) Application number: 21907193.3
(22) Date of filing: 16.12.2021
(51) Int. Cl.: A61K 38/17, A61K 47/68, A61P 25/28, G01N 33/68, C07K 16/28, A61K 39/00

(54) **USE OF TACI PROTEIN**

(30) Priority: 16.12.2020 KR 20200176159
(71) Applicant: Good T Cells, Inc., Seoul 03929 (KR)
(72) Inventor: KIM, Jung Ho, Seoul 04136 (KR); KIM, Beom Seok, Seoul 03929 (KR)
(74) Representative: Pharma Patents International AG
(86) International application number: PCT/KR2021/095127
(87) International publication number: WO 2022/131889

(57) **Abstract**

The present invention relates to a pharmaceutical composition for preventing or treating neurodegenerative diseases or neuroinflammatory diseases comprising, as an active ingredient, antibodies or antigen-binding fragments specific to TACI (transmembrane activator and CAML interactor) protein or fragments thereof, and neurodegenerative diseases or neuroinflammatory diseases can be prevented or treated through the composition.

## Description

### Technical Field

The present invention relates to a use of TACI protein.

### Background Art

Although regulatory T cells (Treg), a subtype of T cells in our body, play an important role of naturally preventing the occurrence of excessive inflammation and immune responses, it is known that the function and number of regulatory T cells are significantly reduced when autoimmune diseases and chronic inflammatory diseases occur. Therefore, in the case of patients with immune diseases and inflammatory diseases, generating many regulatory T cells is the most important and natural treatment. So far, several genes and proteins that exist specifically in regulatory T cells (Treg) have been discovered and announced as follows. Recently, LRIG1 (leucine-rich and immunoglobulin-like domains 1) protein, a transmembrane protein, has been known to be specifically expressed in regulatory T cells (see US20200048343A1).

In order to find genes or proteins that are specifically expressed in regulatory T cells, the researchers conducted flow cytometry (FACS) and the like to identify that TACI protein is specifically expressed in regulatory T cells and associated with the activation and differentiation of regulatory T cells.

### Technical Problem

An object of the present invention is to provide a composition capable of preventing, improving or treating neurodegenerative diseases or neuroinflammatory diseases, and another object of the present invention is to provide a composition capable of diagnosing neurodegenerative diseases or neuroinflammatory diseases, a kit, or a method for providing information for the diagnosis. Furthermore, another object of the present invention is to provide a method for screening drugs for treating neurodegenerative diseases or neuroinflammatory diseases.

Another object of the present invention is to provide a composition capable of preventing, improving or treating cancer, and another object of the present invention is to provide a composition capable of diagnosing cancer, a kit, or a method for providing information for the diagnosis. Furthermore, another object of the present invention is to provide a method for screening drugs for treating cancer.

However, the technical problem to be achieved by the present invention is not limited to the above-mentioned problems, and other problems that are not mentioned will be clearly understood by those of ordinary skill in the art from the following description.

### Solution to Problem

The "TACI (Transmembrane activator and CAML interactor) protein" of the present invention is also known as "TNFRSF13B (tumor necrosis factor receptor superfamily member 13B)"; and is a protein encoded by the TNFRSF13B gene. The TNFRSF13B is a transmembrane protein of the TNF receptor superfamily, which is mainly found on the surface of B cells, and is known to play an important role in the immune system. Such TACI protein can recognize the ligands of APRIL, BAFF, and CAML. In the present invention, TACI protein may consist of the amino acid sequence represented by SEQ ID NO: 1 or SEQ ID NO: 2, but is not limited thereto.

As used herein, nucleic acid molecules comprise all nucleic acid molecules in which the amino acid sequence of the polypeptide provided in the present invention is translated into a polynucleotide sequence as known to those skilled in the art. As a result, various polynucleotide sequences can be generated via open reading frame (ORF), and all of these sequences are encompassed within the nucleic acid molecule of the present invention.

In the present invention, the "vector" is a nucleic acid molecule capable of transporting another nucleic acid linked thereto. One type of vector is a "plasmid", which refers to circular double-stranded DNA into which an additional DNA segment can be ligated. Another type of vector is a phage vector. Yet another type of vector is a viral vector, where an additional DNA segment can be ligated into the viral genome. Certain vectors are capable of autonomous replication in a host cell into which they are introduced (for example, bacterial vectors having a bacterial origin of replication and episomal mammalian vectors). Other vectors (for example, non-episomal mammalian vectors) can be integrated into the genome of a host cell upon introduction into the host cell, and thus are replicated along with the host genome. In addition, certain vectors are capable of directing expression of genes to which they are operatively linked. Such vectors are referred to as "recombinant expression vectors" or simply "expression vectors". In general, expression vectors useful in recombinant DNA techniques are often in the form of plasmids. In the present specification, "plasmid" and "vector" may be used interchangeably as the plasmid is the most commonly used form of vector.

Specific examples of the expression vector in the present invention may be selected from, but are not limited to, the group consisting of commercially widely used pCDNA vectors, F, R1, RP1, Col, pBR322, ToL, Ti vectors; cosmids; phages such as lambda, lambdoid, M143, Mu, p1, P22, Qµµ, T-even, T2, T3, T7; plant viruses. Any expression vector known, to those skilled in the art, as expression vectors can be used in the present invention, and the expression vector is selected depending on the nature of the target host cell. Introduction of a vector into a host cell may be performed by calcium phosphate transfection, viral infection, DEAE-dextran-mediated transfection, lipofectamine transfection, or electroporation. However, the present invention is not limited thereto, and those skilled in the art may adopt and use an introduction method appropriate for the expression vector and the host cell which are used. The vector may preferably contain at least one selection marker. However, the present invention is not limited thereto, and selection can be made using the vector that contains no selection marker, depending on whether or not a product is produced. The selection marker is selected depending on the target host cell, which is done using methods already known to those skilled in the art, and thus the present invention has no limitation thereon.

In order to facilitate purification of the nucleic acid molecule of the present invention, a tag sequence may be inserted into and fused to an expression vector. The tag includes, but is not limited to, hexa-histidine tag, hemagglutinin tag, myc tag, or flag tag, and any tag known to those skilled in the art which facilitates purification can be used in the present invention.

In the present invention, the "host cell" includes individual cells or cell cultures which may be or have been recipients of the vector(s) for incorporation of a polypeptide insert. The host cell includes progeny of a single host cell, and the progeny may not necessarily be completely identical (in morphology or in genomic DNA complement) to the original parent cell due to natural, accidental, or intentional mutation. The host cell includes cells transfected *in vivo* with the polynucleotide(s) herein.

In the present invention, the host cell may include cells of mammalian, plant, insect, fungal or cellular origin, and may be, for example, bacterial cells such as E. coli, Streptomyces, Salmonella typhimurium; fungal cells such as yeast cells and Pichia pastoris; insect cells such as Drosophila and Spodoptera Sf9 cells; animal cells such as Chinese hamster ovary (CHO) cells, SP2/0 (mouse myeloma), human lymphoblastoid, COS, NSO (mouse myeloma), 293T, Bowes melanoma cells, HT-1080, baby hamster kidney (BHK) cells, human embryonic kidney (HEK) cells, or PERC.6 (human retinal cells); or plant cells. However, the host cell is not limited thereto, and any cell known to those skilled in the art which can be used as a host cell line is available.

In the present invention, the neurodegenerative disease or neuroinflammatory disease may be at least one selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, cortical degeneration syndrome, multiple system atrophy, progressive supranuclar palsy, nervous system autoimmune disease, spinocerebellar ataxia, inflammatory and neuropathic pain, cerebrovascular disease, spinal cord injury and tauopathy, but is not limited thereto.

In the present invention, the "immunoglobulin Fc region" refers to a region comprising heavy chain constant region 2 (CH2) and/or heavy chain constant region 3 (CH3), excluding the heavy chain and light chain variable regions of immunoglobulin. The immunoglobulin Fc region may be one component constituting the moiety of the protein conjugate of the present invention.

In the present invention, the immunoglobulin Fc region may comprise a hinge portion in a heavy chain constant region, but is not limited thereto.

In the present invention, the immunoglobulin Fc region may be an extended Fc region comprising part or all of the heavy chain constant region 1 (CH1) and/or light chain constant region 1 (CL1), except for the heavy chain and light chain variable regions of immunoglobulin, as long as it has an effect substantially equal to or improved from that of the natural type. In addition, it may be a region in which a part of a fairly long amino acid sequence corresponding to CH2 and/or CH3 is removed.

In the present invention, the immunoglobulin Fc region may be a dimer of 1) CH1 domain, CH2 domain, CH3 domain and CH4 domain, 2) CH1 domain and CH2 domain, 3) CH1 domain and CH3 domain, 4) CH2 domain and CH3 domain, 5) combination of one or two or more of the CH1 domain, CH2 domain, CH3 domain and CH4 domain with an immunoglobulin hinge region (or part of a hinge region), 6) each domain of heavy chain constant region and light chain constant region, but is not limited thereto.

In the present invention, the immunoglobulin Fc region comprises a natural amino acid sequence as well as a sequence derivative thereof. An amino acid sequence derivative means that one or more amino acid residues in a natural amino acid sequence have a different sequence due to deletion, insertion, non-conservative or conservative substitution, or a combination thereof. For example, in the case of IgG Fc, amino acid resides 214 to 238, 297 to 299, 318 to 322, or 327 to 331 known to be important for binding may be used as suitable sites for modification.

In the present invention, the Fc region may have a variety of derivatives, such as removing a site capable of forming a disulfide bond, removing some amino acids from the N-terminal from the natural Fc, or adding a methionine residue to the N-terminal of the natural Fc. In addition, in order to eliminate the effector function, a complement binding site such as a C1q binding site may be removed, and an antibody dependent cell mediated cytotoxicity (ADCC) site may be removed. Techniques for preparing these sequence derivatives of the immunoglobulin Fc region are disclosed in International Patent Publication Nos. WO 97/34631 and International Patent Publication No. 96/32478.

In the present invention, the protein of the fusion protein can be prepared as a modified Fc derivative according to known methods as long as its activity is not changed as a whole. For example, amino acid exchanges in proteins and peptides which do not entirely alter activity of a molecule are known in the art. The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu and Asp/Gly. In some cases, it is modified by phosphorylation, sulfation, acrylation, glycosylation, methylation, farnesylation, acetylation and amidation.

In the present invention, the Fc derivative exhibits biological activity equivalent to that of the Fc region of the present invention and may have increased structural stability of the Fc region against heat and pH.

In the present invention, the Fc region may be obtained from a natural form isolated from animals such as humans, cows, goats, pigs, mice, rabbits, hamsters, rats, or guinea pigs, or may be a recombinant or derivative thereof obtained from a transformed animal cell or microorganism. Here, the method of obtaining from a natural form may be a method of separating the entire immunoglobulin from the living body of a human or animal, and then processing the proteolytic enzyme. When treated with papain, it is cleaved into Fab and Fc, and when treated with pepsin, it is cleaved into pF'c and F(ab)2. Fc or pF'c may be separated using size-exclusion chromatography or the like. In a more specific embodiment, the Fc region is a recombinant immunoglobulin Fc region obtained by obtaining a human or mouse-derived Fc region from a microorganism.

In the present invention, the immunoglobulin Fc region may be in the form of a natural sugar chain, or in which the sugar chain increased compared to the natural form, in which the sugar chain decreased compared to the natural form, or in which the sugar chain is removed. Conventional methods such as chemical methods, enzymatic methods, and genetic engineering methods using microorganisms may be used to increase or decrease immunoglobulin Fc sugar chains. Here, the immunoglobulin Fc region in which the sugar chain is removed from Fc has significantly reduced binding ability with complement (c1q), and the antibody-dependent cytotoxicity or complement-dependent cytotoxicity is reduced or eliminated, so unnecessary immune responses are not induced *in vivo.* In this regard, a form more suitable for its original purpose as a drug carrier will be referred to as an immunoglobulin Fc region in which sugar chains or removed or non-glycosylated.

In the present invention, the "deglycosylation" refers to the removal of sugars using an enzyme capable of removing sugars from the Fc region, and "aglycosylation" refers to an Fc region produced by a prokaryotic animal, such as Escherichia coli, and not glycosylated.

In the present invention, the immunoglobulin Fc region may be an Fc region derived from IgG, IgA, IgD, IgE or IgM, heavy chain constant region 2 (CH2), heavy chain constant region 3 (CH3), hinge, a fragment thereof, a combination thereof, or a hybrid Fc comprising a combination thereof.

In the present invention, the "combination" refers to the polypeptide encoding a single-chain immunoglobulin Fc region, heavy chain constant region 2 (CH2) or heavy chain constant region 3 (CH3) of the same origin forming a bond with short-chain polypeptides of different origins when forming dimers or multimers. That is, a dimer or multimer can be prepared from two or more fragments selected from an Fc region derived from IgG, IgA, IgM, IgD or IgE, heavy chain constant region 2 (CH2), heavy chain constant region 3 (CH3).

In the present invention, the "hybrid Fc" may be derived from a combination of human IgG subclasses or a combination of human IgD and IgG. In one embodiment, the hybrid Fc may comprise, for example, an IgD hinge region and a CH2 N-terminal region + IgG4 CH2 and CH3 region, and for example, the hybrid Fc form disclosed in Korean Patent Registration No. 0897938 may be equally borrowed and used, and is incorporated herein by reference. In the present invention, in a case where the hybrid Fc binds to a biologically active molecule, polypeptide, or the like, the hybrid Fc has effects of not only increasing a serum half-life of the biologically active molecule, but also increasing the expression level of the polypeptide when a nucleotide sequence encoding the Fc-polypeptide fusion protein is expressed.

As an example of the present invention, the immunoglobulin Fc region may be an Fc region derived from IgG or IgM, which is most abundant in human blood, or may comprise heavy chain constant region 2 (CH2) and heavy chain constant region 3 (CH3) derived from IgG or IgM, and as another example, it may be an Fc region derived from an IgG known to enhance the half-life of a ligand binding protein, or may comprise heavy chain constant region 2 (CH2) and heavy chain constant region 3 (CH3) derived from IgG, and as another example, it may be an Fc region derived from IgG1, IgG2, IgG3, or IgG4, or may comprise heavy chain constant region 2 (CH2) and heavy chain constant region 3 (CH3) derived from IgG1, IgG2, IgG3, or IgG4, and as another example, it may comprise heavy chain constant region 2 (CH2) and heavy chain constant region 3 (CH3) derived from IgG1 or IgG2.

When TACI protein of the present invention or fragment thereof and the Fc region are linked through a linker, the linker may be linked to the N-terminus, C-terminus or the free radical of the Fc fragment, and to the N-terminus, C-terminus or the free radical of TACI protein. When the linker is a peptide linker, linkage can occur at any site. For example, the linker may be linked to the C-terminus of the immunoglobulin Fc region and the N-terminus of TACI protein.

In the present invention, the "linker" may increase the desired activity of TACI protein in a target cell by reducing interference between TACI protein or fragments thereof and the immunoglobulin Fc region in the fusion protein. In addition, the linker may comprise a sequence that can be cleaved by an enzyme that is overexpressed in a tissue or cell having a target disease. In a case where the linker may be cleaved by the overexpressed enzyme as described above, it is possible to effectively prevent activity of a polypeptide from decreasing due to the Fc region.

As an example of the present invention, the linker preferably has 1 to 100 amino acids, but is not limited thereto, and may be any peptide capable of separating TACI protein or fragments thereof and the immunoglobulin Fc region in the fusion protein. The amino acid sequence constituting the linker is not particularly limited, but preferably comprises glycine (G) and serine (S), or comprises them in a repetitive or random pattern. As such, the linker may comprise at least one of a peptide linker represented by GS and a peptide linker represented by GGG, or may comprise the amino acid sequence of (GGGGS)_{N} (N is an integer greater than or equal to 1, preferably an integer of 1 to 20).

In addition, as an example of the linker in the present invention, the linker may be a peptide linker consisting of 33 amino acids located in the 282^{nd} to 314^{th} portion of human albumin, which is most abundantly present in the blood, and preferably a peptide linker consisting of 13 amino acids located in the 292^{nd} to 304^{th} portion of the human albumin. Such portions are portions which are mostly exposed to the outside in three-dimensional structure, and thus have a minimum possibility of inducing an immune response in the body. However, the linker is not limited thereto.

In addition, when the linker and the Fc region are expressed separately and then combined, the linker may be a cross-linking agent known in the art. The cross-linking agent may be, for example, imidoesters comprising disuccinimidyl esters such as 1,1-bis(diazoacetyl)-2-phenylethane, glutaraldehyde, N-hydroxysuccinimide ester such as 4-azidosalicylic acid, 3,3'-dithiobis(succinimidylpropionate), and bifunctional maleimides such as bis-N-maleimido-1,8-octane, but is not limited thereto.

The antibody or antigen-binding fragment of the present invention may maintain or inhibit the function of regulatory T cells by specifically binding to TACI protein or fragments thereof present on the surface of regulatory T cells. In other words, the antibody can suppress signaling by inhibiting regulatory T cells. This means that it can function as an antagonist. Antagonists may specifically bind to a receptor and inhibit the ligand-induced regulatory action of cells by interfering with the interaction between the existing ligand and the receptor. For example, antibodies that block PD-1, CTLA-4, and the like are all antagonistic antibodies that can block immune resistance of tumors by immune checkpoint proteins.

In addition, according to the research result (Baruch K, Rosenzweig N, Kertser A, Deczkowska A, Sharif AM, Spinrad A, Tsitsou-Kampeli A, Sarel A, Cahalon L, Schwartz M. 2015. Breaking immune tolerance by targeting Foxp3(+) regulatory T cells mitigates Alzheimer's disease pathology. Nat Commun 6: 7967), which found that reversal of the phenomenon of cognitive decline occurs and the neurogenic inflammatory response is alleviated in the case of a temporary deficiency of regulatory T cells or suppression of the activity of regulatory T cells in the 5XFAD AD mouse model, the antibody or antigen-binding fragment of the present invention can prevent or treat neurodegenerative disease or neuroinflammatory disease by specifically binding to TACI protein or fragments thereof present on the surface of regulatory T cells to maintain or inhibit the function of regulatory T cells.

The antibody of the present invention has the ability to bind to TACI protein or fragments thereof as a full-length antibody or a part of an antibody, and comprises all antibody fragments that binds competitively with the binding molecule of the present invention to the antigenic determining region (epitope) of TACI protein or fragments thereof.

In the present invention, the "antibody" refers to a protein molecule which serves as a receptor that specifically recognizes an antigen, including an immunoglobulin molecule that is immunologically reactive with a particular antigen. For the purpose of the present invention, the antigen may be a TACI protein or fragments thereof present on the surface of regulatory T cells, preferably regulatory T cells in which the inhibitory ability of effector T cells is activated. Preferably, using TACI protein or fragments thereof as an example, the antibody may specifically recognize a leucine rich region or an immunoglobulin-like domain of an extracellular domain, but is not limited thereto.

In the present invention, the "immunoglobulin" has a heavy chain and a light chain, and each of the heavy chain and the light chain comprises a constant region and a variable region. The variable region of each of the light chain and the heavy chain contains three hypervariable regions called complementarity determining regions (hereinafter referred to as "CDRs") and four framework regions. The CDRs primarily serve to bind to an epitope on an antigen. The CDRs of each chain are typically referred to as CDR1, CDR2, and CDR3 sequentially starting from the N-terminus, and are also distinguished by the chain where particular CDRs are located.

In the present invention, the "antibody" includes three variable regions and four framework regions called the complementarity determining regions of the present invention. The CDRs primarily serve to bind to an epitope on an antigen. The CDRs of each chain are typically referred to as CDR1, CDR2, and CDR3 sequentially starting from the N-terminus, and are also distinguished by the chain where particular CDRs are located.

In the present invention, the "full-length antibody" has a structure with two full-length light chains and two full-length heavy chains in which each light chain is linked to a heavy chain by a disulfide bond, and includes IgA, IgD, IgE, IgM, and IgG. The IgG includes, as subtypes thereof, IgG1, IgG2, IgG3, and IgG4.

In the present invention, the "antigen-binding fragment" refers to a fragment with an antigen-binding function, and examples of antigen-binding fragments comprise (i) a Fab fragment consisting of a light chain variable region (VL), a heavy chain variable region (VH), a light chain constant region (CL), and a heavy chain first constant region (CH1); (ii) a Fd fragment consisting of the VH and CH1 domains; (iii) a Fv fragment consisting of the VL and VH domains of a single antibody; (iv) a dAb fragment consisting of the VH domain; (v) isolated CDR regions; (vi) F(ab')2 fragment, which is a bivalent fragment comprising two linked Fab fragments; (vii) a single-chain Fv molecule (scFv) linked by a peptide linker that links the VH domain and the VL domain to form an antigen-binding site; (viii) bispecific single-chain Fv dimers; (ix) diabodies, which are multivalent or multispecific fragments produced by gene fusion, and the like. The antigen-binding fragment may be a Fab or F(ab')2 fragment when using a proteolytic enzyme, for example, papain or pepsin, and may be produced through genetic recombination technology.

In the present invention, the antibody may be a monoclonal antibody, a polyclonal antibody, a chimeric antibody, a humanized antibody, a bivalent, a bispecific molecule, a minibody, a domain antibody, a bispecific antibody, an antibody mimic, a unibody, a diabody, a triabody, a tetrabody or a fragment thereof, but is not limited thereto.

In the present invention, the "monoclonal antibody" refers to an antibody molecule of a single molecular composition which is obtained from substantially the same antibody population, and exhibits single binding specificity and affinity for a particular epitope.

In the present invention, the "chimeric antibody" is an antibody which is obtained by recombination of a variable region of a mouse antibody and a constant region of a human antibody, and has a greatly improved immune response as compared with the mouse antibody.

In the present invention, the "humanized antibody" refers to an antibody obtained by modifying a protein sequence of an antibody derived from a non-human species so that the protein sequence is similar to an antibody variant naturally produced in humans. For example, the humanized antibody may be prepared as follows. Mouse-derived CDRs may be recombined with a human antibody-derived FR to prepare a humanized variable region, and the humanized variable region may be recombined with a constant region of a preferred human antibody to prepare a humanized antibody.

In the present invention, the "binding" or "specific binding" refers to the affinity of the antibody or antibody composition for an antigen. "Specific binding" in antigen-antibody binding can typically be distinguished from non-specific background binding if the dissociation constant (Kd) is less than 1×10⁻⁵M or less than 1×10⁻⁶M or less than 1×10⁻⁷M. Specific binding can be detected by methods known in the art, such as ELISA, surface plasmon resonance (SPR), immunoprecipitation, coprecipitation, and the like, and comprise appropriate controls to distinguish between non-specific and specific binding.

Antibodies or antigen-binding fragments of the present invention may exist as multimers such as dimers, trimers, tetramers, and pentamers that comprise at least a portion of the antigen-binding ability of monomers. These multimers also include homomultimers or heteromultimers. Since antibody multimers contain multiple antigen-binding sites, they have superior antigen-binding ability compared to monomers. Multimers of antibodies facilitate the production of multifunctional (bifunctional, trifunctional, tetrafunctional) antibodies.

In the present invention, the term "multifunctional" refers to an antibody or antigen-binding fragment having two or more activities or functions (such as antigen-binding ability enzyme activity, ligand or receptor binding ability), and for example, the antibody of the present invention may be coupled to a polypeptide with enzymatic activity, such as luciferase, acetyltransferase, galactosidase, and the like. Multifunctional antibodies also include antibodies in the form of multivalent or multispecific (bispecific, trispecific, etc.).

In the present invention, the "antibody-drug conjugate (ADC)" refers to a form in which the drug and the antibody are chemically linked to each other without degrading biological activity of the antibody and the drug. In the present invention, the antibody-drug conjugate denotes a form in which the drug is bound to an amino acid residue at the N-terminus of the heavy and/or light chain of the antibody, and specifically, a form in which the drug is bound to an α-amine group at the N-terminus of the heavy and/or light chain of the antibody.

In the present invention, the "drug" may mean any substance having a certain biological activity for a cell, which is a concept including DNA, RNA, or a peptide. The drug may be in a form which contains a reactive group capable of reacting and crosslinking with an α-amine group, and also includes a form which contains a reactive group capable of reacting and crosslinking with an α-amine group and to which a linker is linked.

In the present invention, examples of the reactive group capable of reacting and crosslinking with the α-amine group are not particularly limited in terms of type as long as the reactive group can react and crosslink with an α-amine group at the N-terminus of a heavy or light chain of an antibody. The reactive group includes all types of groups known in the art which react with an amine group. The reactive group may, for example, be any one of isothiocyanate, isocyanate, acyl azide, NHS ester, sulfonyl chloride, aldehyde, glyoxal, epoxide, oxirane, carbonate, aryl halide, imidoester, carbodiimide, anhydride and fluorophenyl ester, but is not limited thereto.

In the present invention, the drug may include any drug regardless of type as long as the drug can treat brain and nervous system diseases or cancer, in particular, neurodegenerative diseases or neuroinflammatory diseases or cancer.

In the present invention, the "prevention" can include, without limitation, any act of blocking symptoms, or suppressing or delaying the symptoms, using the pharmaceutical composition of the present invention.

In the present invention, the "treatment" and "improvement" may include, without limitation, any act capable of improving or beneficially altering the symptoms of a disease by examining the pharmaceutical composition of the present invention.

In the present invention, the pharmaceutical composition may be characterized by being in the form of capsules, tablets, granules, injections, ointments, powders, or beverages, and the pharmaceutical composition may be characterized by being targeted to humans.

The pharmaceutical composition of the present invention may be formulated in the form of oral preparations such as powders, granules, capsules, tablets, and aqueous suspensions, preparations for external use, suppositories, and sterile injectable solutions, respectively, according to conventional methods, and used. However, the pharmaceutical composition is not limited thereto. The pharmaceutical composition of the present invention may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The preparations of the pharmaceutical composition of the present invention may be prepared in various ways by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, as examples of carriers, diluents, or excipients suitable for making preparations, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like may be used. In addition, a filler, an anti-coagulant, a lubricant, a wetting agent, a fragrance, an emulsifier, a preservative, and the like may further be included.

The route of administration of the pharmaceutical composition according to the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

In the present invention, the "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including activity of a certain compound used, the patient's age, body weight, general health status, sex, diet, frequency of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 50 mg/kg or 0.001 to 50 mg/kg, per day. Administration may be made once a day or several times a day. The dose is not intended to limit the scope of the invention in any way. The pharmaceutical composition according to the present invention may be formulated in the form of pills, sugar-coated tablets, capsules, liquids, gels, syrups, slurries, or suspensions.

In the present invention, the agent for measuring the expression level of TACI protein or fragments thereof is not particularly limited, but, for example, may comprise at least one of those selected from the group consisting of antibodies, oligopeptides, ligands, peptide nucleic acids (PNAs) and aptamers that specifically bind to the protein.

In the present invention, the "antibody" refers to a substance that specifically binds to an antigen and causes an antigen-antibody reaction. For the purposes of the present invention, the antibody refers to an antibody that specifically binds to TACI protein or extracellular domain thereof. The antibodies of the present invention comprise all of polyclonal antibodies, monoclonal antibodies and recombinant antibodies. Such antibodies can be readily prepared using techniques well known in the art. For example, polyclonal antibodies can be produced by a method well known in the art, which comprises the process of obtaining serum comprising antibodies by injecting an antigen of a biomarker protein into an animal and collecting blood from the animal. Such monoclonal antibodies can be prepared from any animal such as goat, rabbit, sheep, monkey, horse, pig, cow, dog, and the like. In addition, monoclonal antibodies can be prepared using hybridoma methods well known in the art, or phage antibody library technology. The antibody prepared by the above method may be separated and purified using methods such as gel electrophoresis, dialysis, salt precipitation, ion exchange chromatography, and affinity chromatography. In addition, the antibodies of the present invention comprise functional fragments of antibody molecules, as well as complete forms with two full-length light chains and two full-length heavy chains. A functional fragment of an antibody molecule means a fragment with at least an antigen-binding function, and includes Fab, F(ab'), F(ab')2, and Fv.

In the present invention, the "PNA (peptide nucleic acid)" refers to an artificially synthesized polymer similar to DNA and RNA, and was first introduced in 1991 by Professors Nielsen, Egholm, Berg, and Buchardt. While DNA has a phosphate-ribose backbone, PNA has a repeated N-(2-aminoethyl)-glycine backbone linked by peptide bonds, which greatly increases the binding force and stability to DNA or RNA, and is therefore used in molecular biology, diagnostic assays, and antisense therapies.

In the present invention, the "aptamer" is an oligonucleic acid or peptide molecule.

The agent for measuring the expression level of the gene encoding TACI or fragments thereof of the present invention may comprise at least one selected from the group consisting of a primer, a probe, and an antisense nucleotide that specifically binds to the gene.

In the present invention, the "primer" refers to a fragment that recognizes a target gene sequence, and consists of a forward and reverse primer pair, but preferably provide analysis results with specificity and sensitivity. High specificity can be imparted when the primers amplify the target gene sequence containing complementary primary binding sites while not causing non-specific amplification because the nucleic acid sequence of the primer is inconsistent with the non0target sequence present in the sample.

In the present invention, the "probe" refers to a substance that can specifically bind to a target substance to be detected in a sample, and a substance that can specifically confirm the presence of a target substance in a sample through binding. Given probes are commonly used in the art, the type of probe is not limited, but preferably can be peptide nucleic acid (PNA), locked nucleic acid (LNA), peptide, polypeptide, protein, RNA, or DNA, with PNA being the most preferred. More specifically, the probe is a biomaterial that includes those derived from or similar to those derived from living organisms or those manufactured *in vitro,* for example, enzymes, proteins, antibodies, microorganisms, animal and plant cells and organs, nerve cells, DNA, and RNA. DNA may comprise cDNA, genomic DNA and oligonucleotides, while RNA may comprise genomic RNA, mRNA, oligonucleotides, while proteins may comprise antibodies, antigens, enzymes, peptides, and the like.

In the present invention, the "LNA (locked nucleic acids)" refers to a nucleic acid analog comprising a 2'-O, 4'-C methylene bridge. LNA nucleosides comprise the common nucleic acid bases of DNA and RNA, and can base pair according to the Watson-Crick base pairing rules. However, because of the locking of molecules due to the methylene bridge, LNA is unable to form an ideal shape in Watson-Crick base pairing. When LNAs are comprised in the oligonucleotides of DNA or RNA, LNAs can more rapidly pair with complementary nucleotide chains to increase the stability of the double helix.

In the present invention, the "antisense" refers to oligomers with a sequence of nucleotide bases and an inter-subunit backbone, in which the antisense oligomer is hybridized with a target sequence in RNA by Watson-Crick base pair formation, allowing the formation of mRNA and RNA:oligomer heteroduplexes in the target sequence. Oligomers can have a sequence that has exactly or near complementarity to the target sequence.

Information regarding TACI protein or the gene that encodes it is known, and thus those skilled in the art can readily design primers, probes or antisense nucleotides that selectively bind to the gene that encodes the protein based on such information.

The kit of the present invention may be an RT-PCR kit, a DNA chip kit, an ELISA kit, a protein chip kit, a rapid kit, or a multiple reaction monitoring (MRM) kit, but is not limited thereto.

The diagnostic kit of the present invention may further comprise one or more other component compositions, solutions or devices suitable for assay methods.

For example, the diagnostic kit of the present invention may further comprise essential elements required for performing reverse transcription polymerase reaction. The kit for reverse transcription polymerase reaction comprises a pair of primers specific for a gene encoding a marker protein. The primer is a nucleotide having a sequence specific to a nucleic acid sequence of the gene, and may have a length of about 7 bp to 50 bp, more preferably about 10 bp to 30 bp. The kit may also comprise a primer specific for a nucleic acid sequence of a control gene. In addition to those mentioned above, the kit for reverse transcription polymerase reaction may comprise test tubes or other suitable containers, reaction buffers (with varying pH and magnesium concentrations), deoxynucleotides (dNTPs), enzymes such as Taq-polymerase and reverse transcriptase, DNase and RNase inhibitors, DEPC-treated water, sterilized water, and the like.

In addition, the diagnostic kit of the present invention may comprise essential elements required for performing a DNA chip assay. The DNA chip kit may comprise a substrate to which a cDNA or oligonucleotide corresponding to a gene or a fragment thereof is attached; reagents, agents, and enzymes for preparing a fluorescence-labeled probe; and the like. The substrate may also comprise a cDNA or oligonucleotide corresponding to a control gene or a fragment thereof.

In addition, the diagnostic kit of the present invention may comprise essential elements required for performing ELISA. The ELISA kit comprises an antibody specific for the marker protein. The antibodies are antibodies with high specificity and affinity for the marker protein and little cross-reactivity with other proteins, and include monoclonal antibodies, polyclonal antibodies, or recombinant antibodies. The ELISA kit may also include an antibody specific for a control protein. In addition to those mentioned above, the ELISA kit may also comprise reagents capable of detecting bound antibodies, for example, labeled secondary antibodies, chromophores, enzymes (which are, for example, conjugated with an antibody) and substrates thereof, and other substances capable of binding to antibodies.

According to another embodiment of the present invention, it relates to a method for providing information for diagnosing a neurodegenerative disease or a neuroinflammatory disease comprising the step of measuring the expression level of TACI protein or fragments thereof, or a gene encoding the same, in a biological sample isolated from a target individual.

In the present invention, the "target individual" refers to an individual or whom it is uncertain whether a neurodegenerative disease or a neuroinflammatory disease has developed and who has a high probability of developing the disease.

In the present invention, the "biological sample" refers to any material, biological fluid, tissue or cell obtained or derived from an individual, and may, for example, comprise whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluids, meningeal fluids, amniotic fluids, glandular fluids, pancreatic fluids, lymph fluids, pleural fluid, nipple aspirate, bronchial aspirate, synovial fluids, joint aspirate, organ secretions, cells, cell extracts or cerebrospinal fluids, but is not limited thereto.

TACI protein or fragments thereof of the present invention may be expressed on the surface of regulatory T cells, preferably TIL regulatory T cells or activated regulatory T cells, more preferably regulatory T cells in which the inhibitory ability of effector T cells is activated.

In the method for providing information for diagnosing a neurodegenerative disease or a neuroinflammatory disease, descriptions of the agent for measuring the expression level of the gene encoding TACI or fragments thereof, or the gene encoding the same, are the same as those described in the diagnostic composition, and thus descriptions thereof are omitted to avoid excessive redundancy.

In the present invention, examples of the method for measuring or comparatively analyzing the expression level of TACI protein or fragments thereof include, but are not limited to, protein chip assay, immunoassay, ligand binding assay, matrix assisted laser desorption/ionization time of flight mass spectrometry (MALDI-TOF), surface enhanced laser desorption/ionization time of flight mass spectrometry (SELDI-TOF), radioimmunoassay, radial immunodiffusion, ouchterlony immunodiffusion, rocket immunoelectrophoresis, immunohistochemical staining, complement fixation assay, two-dimensional electrophoresis, liquid chromatography-mass spectrometry (LC-MS), liquid chromatography-mass spectrometry/mass spectrometry (LC-MS/MS), Western blotting, and enzyme linked immunosorbent assay (ELISA).

In the process of confirming the presence and expression level of the gene encoding TACI protein or fragments thereof of the present invention, analysis methods for measuring the expression level of the gene include reverse transcription polymerase chain reaction (RT-PCR), competitive reverse transcription polymerase chain reaction (competitive RT-PCR), real-time RT-PCR, RNase protection assay (RPA), Northern blotting or DNA chip, but is not limited thereto.

The isolated biological sample of the present invention may be a biological sample isolated from a subject with or without a neurodegenerative disease or a neuroinflammatory disease. Specifically, it refers to any material, biological fluid, tissue or cell obtained from or derived from the subject, and comprises, as an example, whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluids, meningeal fluids, amniotic fluids, glandular fluids, pancreatic fluids, lymph fluids, pleural fluid, nipple aspirate, bronchial aspirate, synovial fluids, joint aspirate, organ secretions, cells, cell extracts or cerebrospinal fluids, but is not limited thereto.

TACI protein or fragments thereof of the present invention may be expressed on the surface of regulatory T cells, preferably TIL regulatory T cells or activated regulatory T cells, more preferably regulatory T cells in which the inhibitory ability of effector T cells is activated.

In the present invention, the test substance comprises any substance, molecule, element, compound, entity, or a combination thereof. Examples include, but are not limited to, proteins, polypeptides, small organic molecules, polysaccharides, polynucleotides, and the like. It may also be a natural product, a synthetic compound or a combination of two or more substances.

In the present invention, "cancer" refers to or represents a physiological condition typically characterized by unregulated cell growth in mammals. In the present invention, the cancer to be prevented, improved, or treated may be a solid tumor formed by abnormal cell growth in a solid organ, and depending on the site of the solid organ, it may be gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer and the like, and may preferably be melanoma, but is not limited thereto.

According to another embodiment of the present invention, there is provided a pharmaceutical composition for prevention or treatment of cancer comprising, as an active ingredient, regulatory T cells comprising TACI protein or fragments thereof, or a gene encoding the same.

In the present invention, the antibody specific to TACI protein or fragments thereof may be a C-9 antibody (mouse anti TACI antibody). TACI protein or fragments thereof may be present in regulatory T cells.

Cancer cells are widely believed to express immunogenic antigens capable of inducing an effective immune response against tumor formation. Additionally, the tumor microenvironment is rich in components that can trigger TLR signaling to activate anti-tumor responses. This means that, in the early stages of the disease, cancer cells may have a chance to be recognized and rejected by the immune system, which exerts both host-protective and tumor-modeling actions against developing tumors. Nonetheless, cancer cells have many negative regulatory mechanisms to evade immune surveillance that induce immune tolerance against cancer cells by increasing the secretion of inhibitory cytokines and expressing inhibitory molecules, such as down-regulation of MHC molecules or antigen processing and presentation machinery. Thus, cancer patients are often considered to have poor immunity. Therefore, there is still a need to develop agents or therapies for reversal of cancer-related immune suppression. On the other hand, antibody-based cancer therapy has potentially high specificity and low side effects compared to conventional drugs. The reason for this being that antibodies can distinguish between normal cells and neoplastic cells and that their mode of action relies on less toxic immunological anti-tumor mechanisms such as the recruitment and complementary activation of cytotoxic immune cells.

Targets for antibody-based therapy must have specific properties that are the basis for adequate differentiation between normal and neoplastic cells. It is needless to say that, in developing effective and safe antibody therapies, targets that are exclusively restricted to tumor cells or completely undetectable on normal tissue are ideal. In another aspect, high levels of overexpression may underlie the therapeutic window, and low side effects exemplified by human epidermal growth factor receptor type 2 (HER-2) as a result of gene amplification make it a good target for the antibody Trastuzumab (Herceptin).

Other targets of antibodies already approved for tumor therapy or in clinical development have unique properties that are not based on numerical overexpression of the target molecule on tumor cells. In the case of an antibody against the proteoglycan MUC-1, the peptide repeat epitope present in the backbone of the target is underglycosylated in the tumor cell and changed to its normal counterpart. For the antibodies against CD20 (rituximab), CD52 (campath-1H) and CD22 (epratuzumab), the antibody targets show comparable expression levels on tumor cells and normal lymphocytes. In this regard, clearance of normal cells by the antibody can be tolerated since target-negative stem cells restore normal lymphocyte repertoire. Another example of the different accessibility of antibody targets is carcinoembryonic antigen (CEA) and carbonic anhydrase IX (CA9). Both of these antigens are expressed in the normal epithelium of the colon and kidney, respectively. However, cytotoxic antibodies are well tolerated because radiolabeled imaging antibodies distinguish between tumor and normal tissues. This is probably because the expression of CA9 and CEA is confined to the luminal side of normal epithelial tissue, which is not accessible to IgG antibodies. Antigenic epithelial cell adhesion molecules (Ep-CAM) also fall into this category. As a homologous cell adhesion molecule to epithelial cells, it is located in the intercellular space. Interestingly, while high affinity anti-Ep CAM antibodies are highly toxic, moderate affinity antibodies are well tolerated. This suggests not only the accessibility of the Ep-CAM target to normal cells, but also that the kinetics of antibody binding can open a new therapeutic window. Although 8 antibodies have been approved for treating neoplastic diseases, most of these are against lymphoma and leukemia. Only 3 monoclonal antibodies, namely Herceptin, Avastin, and Erbitux, are responsive to the types of solid cancers that account for more than 90% of cancer mortality. mAbs recognized with substantial remaining medical need and significant clinical advantages are already available, and their significant commercial success also provided motivation for the development of new innovative approaches well-balanced in developing antibody-based therapies and enhancing their efficacy for different groups of patients. One of the challenges to be mastered regarding the emergence of upgraded next-generation antibody-based cancer therapies is the selection of appropriate target molecules, which are key factors in favorable toxicity/efficacy profiles.

Current antibodies available for treatment of solid cancers do not fully exert the accumulated power of the mode of action inherent in antibody molecules due to their targeted expressed on normal tissues. For example, Her2/neu, the target for Herceptin, is expressed in many normal human tissues including cardiac muscle. As a result, Herceptin is designed to reduce immunity and cannot be administered at maximally effective doses because otherwise unacceptable toxicity would result. This "potentially blunting action" limits Herceptin's therapeutic efficacy.

In addition to suppression of expression in normal tissues associated with toxicity, it is a preferable feature of an ideal antibody target to exhibit tumor-promoting functions while allowing strong and high expression on the surface of tumor cells.

In the present invention, the "transcription factor" refers to a number of proteins that bind to DNA and regulate mRNA transcription. A large number of transcription factors present inside and outside the cell nucleus play a role in regulating gene expression so that cells with the same gene can perform different functions. It is known that transcription factors promote or inhibit gene expression depending on the transcription factor.

In one embodiment of the present invention, there is provided a pharmaceutical composition for prevention or treatment of neurodegenerative diseases or neuroinflammatory diseases comprising an antibody or antigen-binding fragment specific to TACI (transmembrane activator and CAML interactor) protein or fragments thereof as an active ingredient. In this embodiment, there is provided a pharmaceutical composition for prevention or treatment of neurodegenerative diseases or neuroinflammatory diseases wherein the antibody specific to TACI protein or fragments thereof is a human or mouse-derived antibody, a pharmaceutical composition wherein TACI protein or fragments thereof is present in regulatory T cells, a pharmaceutical composition wherein the neurodegenerative diseases or neuroinflammatory diseases are at least one selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, cortical degeneration syndrome, multiple system atrophy, progressive supranuclar palsy, nervous system autoimmune disease, spinocerebellar ataxia, inflammatory and neuropathic pain, cerebrovascular disease, spinal cord injury and tauopathy, and a pharmaceutical composition wherein the pharmaceutical composition is injected into the body through a route selected from oral, intravenous, intramuscular, intraarterial, intramedullary, intradural, intracardiac, transdermal, subcutaneous, intraperitoneal, intranasal, intestinal, topical, sublingual, or rectal route.

In one embodiment of the present invention, there is provided a pharmaceutical composition for prevention or treatment of neurodegenerative diseases or neuroinflammatory diseases comprising, as an active ingredient, an antibody-drug conjugate (ADC) comprising an antibody or antigen-binding fragment specific to TACI (transmembrane activator and CAML interactor) protein or fragments thereof, or drugs. In this embodiment, there is provided a pharmaceutical composition for prevention or treatment of neurodegenerative diseases or neuroinflammatory diseases wherein the antibody specific to TACI protein or fragments thereof is a human or mouse-derived antibody, a pharmaceutical composition wherein TACI protein or fragments thereof is present in regulatory T cells, a pharmaceutical composition wherein the neurodegenerative diseases or neuroinflammatory diseases are at least one selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, cortical degeneration syndrome, multiple system atrophy, progressive supranuclar palsy, nervous system autoimmune disease, spinocerebellar ataxia, inflammatory and neuropathic pain, cerebrovascular disease, spinal cord injury and tauopathy, and a pharmaceutical composition wherein the pharmaceutical composition is injected into the body through a route selected from oral, intravenous, intramuscular, intraarterial, intramedullary, intradural, intracardiac, transdermal, subcutaneous, intraperitoneal, intranasal, intestinal, topical, sublingual, or rectal route.

In one embodiment of the present invention, there is provided a diagnostic composition for neurodegenerative diseases, neuroinflammatory diseases, or cancer comprising an agent for measuring the expression level of TACI (transmembrane activator and CAML interactor) protein, fragments thereof, or a gene encoding the same. In this embodiment, there is provided a diagnostic composition wherein the agent for measuring the expression level of TACI protein or fragments thereof comprises at least one selected from the group consisting of antibodies, oligopeptides, ligands, peptide nucleic acids (PNAs) and aptamers that specifically bind to the protein or fragments thereof, and a diagnostic composition wherein the agent for measuring the expression level of the gene encoding TACI protein or fragments thereof comprises at least one selected from the group consisting of primers, probes and antisense nucleotides that specifically bind to the gene.

In one embodiment of the present invention, there is provided a diagnostic kit for neurodegenerative diseases, neuroinflammatory diseases, or cancer comprising the diagnostic composition.

In one embodiment of the present invention, there is provided a method for providing information for the diagnosis of neurodegenerative diseases, neuroinflammatory diseases, or cancer comprising the step of measuring the expression level of TACI (transmembrane activator and CAML interactor) protein or fragments thereof, or genes encoding the same, in a biological sample isolated from a target individual. In this embodiment, there is provided a method for providing information wherein the biological sample comprises whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluids, meningeal fluids, amniotic fluids, glandular fluids, pancreatic fluids, lymph fluids, pleural fluid, nipple aspirate, bronchial aspirate, synovial fluids, joint aspirate, organ secretions, cells, cell extracts or cerebrospinal fluids, a method for providing information wherein the method of measuring the expression level of TACI protein or fragments thereof is performed by protein chip assay, immunoassay, ligand binding assay, matrix assisted laser desorption/ionization time of flight mass spectrometry (MALDI-TOF), surface enhanced laser desorption/ionization time of flight mass spectrometry (SELDI-TOF), radioimmunoassay, radial immunodiffusion, ouchterlony immunodiffusion, rocket immunoelectrophoresis, immunohistochemical staining, complement fixation assay, two-dimensional electrophoresis, liquid chromatography-mass spectrometry (LC-MS), liquid chromatography-mass spectrometry/mass spectrometry (LC-MS/MS), Western blotting, or enzyme linked immunosorbent assay (ELISA), a method for providing information wherein the method of measuring the expression level of the gene encoding TACI protein or fragments thereof is performed by reverse transcription polymerase chain reaction (RT-PCR), competitive reverse transcription polymerase chain reaction (competitive RT-PCR), real-time RT-PCR, RNase protection assay (RPA), Northern blotting or DNA chip, and a method for providing information that predicts a high probability of developing a neurodegenerative disease, neuroinflammatory disease, or cancer when the expression level of TACI protein, fragments thereof, or the gene encoding the same, measured from the biological sample, is reduced when compared to the normal control group.

In one embodiment of the present invention, there is provided a method for screening drugs for treating neurodegenerative diseases, neuroinflammatory diseases, or cancer comprising the step of measuring the expression level of TACI (transmembrane activator and CAML interactor) protein or fragments thereof, or a gene encoding the same, in a separated biological sample, the step of contacting the biological sample with a test substance, and the step of measuring the expression level of TACI (transmembrane activator and CAML interactor) protein or fragments thereof, or a gene encoding the same, in a separated biological sample after contact with the test substance.

In one embodiment of the present invention, there is provided a pharmaceutical composition for prevention or treatment of cancer comprising, as an active ingredient, antibodies or antigen-binding fragments specific to TACI (transmembrane activator and CAML interactor) protein or fragments thereof. In this embodiment, there is provided a pharmaceutical composition for prevention or treatment of cancer wherein the antibodies specific to TACI protein or fragments thereof are human or mouse-derived antibodies, a pharmaceutical composition wherein TACI protein or fragments thereof is present in regulatory T cells, a pharmaceutical composition wherein the cancer is a solid cancer, a pharmaceutical composition wherein the cancer is gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer, and a pharmaceutical composition wherein the pharmaceutical composition is injected into the body through a route selected from oral, intravenous, intramuscular, intraarterial, intramedullary, intradural, intracardiac, transdermal, subcutaneous, intraperitoneal, intranasal, intestinal, topical, sublingual, or rectal route.

In one embodiment of the present invention, there is provided a pharmaceutical composition for preventing or treating cancer comprising, as an active ingredient, an antibody-drug conjugate (ADC) comprising antibodies or antigen-binding fragments specific to TACI (transmembrane activator and CAML interactor) protein or fragments thereof, and drugs, a pharmaceutical composition for preventing or treating cancer wherein antibodies specific to TACI protein or fragments thereof are human or mouse-derived antibodies, a pharmaceutical composition wherein TACI protein or fragments thereof is present in regulatory T cells, a pharmaceutical composition wherein the cancer is a solid cancer, a pharmaceutical composition wherein the cancer is gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer, and a pharmaceutical composition wherein the pharmaceutical composition is injected into the body through a route selected from oral, intravenous, intramuscular, intraarterial, intramedullary, intradural, intracardiac, transdermal, subcutaneous, intraperitoneal, intranasal, intestinal, topical, sublingual, or rectal route.

### Advantageous Effects of Invention

In the present invention, TACI protein or fragments thereof are specifically expressed in regulatory T cells in which the inhibitory ability of effector T cells is activated, and it can be used as a new target for diagnosis or treatment of neurodegenerative diseases, neuroinflammatory diseases or cancer.

Neurodegenerative diseases, neuroinflammatory diseases or cancer can be diagnosed or treated by inhibiting the differentiation of iTreg cells by inhibiting the activation of T cells during differentiation process of regulatory T cells using antibodies or antigen-binding fragments specifically bind to TACI protein or fragments thereof.

### Brief Description of Drawings

FIG. 1, FIGS. 2a and 2b illustrate that TACI protein according to an embodiment of the present invention is highly expressed in regulatory T cells.
FIGS. 3a to 3b illustrate that TACI expression on the surface of Treg cells is induced by the negative immune modulator TGF-B, as it can be seen that the expression of TACI increases as the concentration of TGF-B increases according to an embodiment of the present invention.
FIGS. 4a to 4b illustrate that TACI expression is correlated with Foxp3 expression, as it can be seen that TACI expression is increased in iTreg cells of mice with high Foxp3 expression according to an embodiment of the present invention.
FIG. 5 illustrates that the C-9 antibody suppresses differentiation into iTreg cells by suppressing its activity during the process of iTreg differentiation, as it can be seen that the expression of CD69 protein was low when the C-9 (see mouse monoclonal Ab raised against amino acids 42-293 mapping at the C-terminus of TACI of human origin, Santa Cruz [Cat No. sc-365253]) antibody was administered according to an embodiment of the present invention.
FIG. 6 illustrates that the C-9 antibody suppresses differentiation into iTreg cells by suppressing its activity during the process of iTreg differentiation, as it can be seen that the expression of transcription factors is low in Treg cells when the C-9 antibody was administered according to an embodiment of the present invention.
FIG. 7 illustrates that the C-9 antibody suppresses differentiation into iTreg cells by suppressing its activity during the process of iTreg differentiation, as it can be seen that the expression of Foxp3 protein in Treg cells is low when the C-9 antibody as administered according to an embodiment of the present invention.

### Detailed Description of Invention

Hereinafter, the present invention will be described in more detail by way of examples. However, the examples are only for illustrating the present invention, and the contents of the present invention are not limited by these examples.

### [Example 1] Confirmation of TACI expression level by cell

### [1-1] Differentiation protocol for each T-cell subset

After separating the spleen from mice, it was placed in a 60Ψ dish containing 3ml of PBS, rinsed, and then transferred to another 60Ψ dish with 3ml of PBS. After grinding the spleen with the lid of the e-tube, the remaining solution except the tissue was transferred to a 50 ml conical tube, and the lid of the e-tube was washed with PBS and also put in the tube. After centrifugation at 1500 rpm for 5 minutes, the remaining PBS except about 1 ml was aspirated, and the pellet was dissolved with RBC hemolysis buffer (5ml per mouse). The dissolved cells were filtered on a cell strainer in a new 50 ml conical tube, washed with PBS and adjusted to a total volume of 50 ml, and then the number of cells was counted. (Approximately 107 to 108 cells were observed per mouse.) After centrifugation at 1500 rpm for 5 minutes, 400 µl (~30%) of MACS buffer and 100 µl (~30%) of CD4+ cocktail was added per 108 cells and placed in the refrigerator for 10 minutes (tapping for 15 seconds every 1 minute). 300 µl of MACS buffer (~30%) and 200 µl of anti-biotin (~30%) were added to the conical above and placed in the refrigerator for 15 minutes (tapping for 15 seconds every 1 minute). After adjusting to 20 ml with MACS buffer, centrifugation was performed at 1200 rpm for 10 minutes (simultaneously, the LD column was filled with 3 ml of MACS buffer). After aspirating except only 1 ml, it was dissolved with 3 ml of MACS buffer, and after placing a paper cup, ice, and a 50 ml conical under the LD column, it was poured onto the cell filter (repeated twice more). After adjusting to 20 ml with MACS buffer, centrifugation was performed at 1200 rpm for 10 minutes. After aspirating except only 1 ml, 800 µl (~30%) of MACS buffer and 200 µl (~30%) of anti-CD62L+ were added per 108 cells and placed in the refrigerator for 15 minutes (tapping for 15 seconds every 1 minute). After adjusting to 20 ml with MACS buffer, centrifugation was performed at 1200 rpm for 10 minutes (fill LS column with 3 ml of MACS buffer when 5 minutes are left). After aspirating except only 1 ml, it was dissolved with 3 ml of MACS buffer, and after placing a paper cup under the LS column, it was poured onto the cell filter (repeated twice more). The LS column was placed on a 15 ml conical tube and 3 ml of MACS buffer was added, and then the beads were pushed away from the LS column using a cylinder. After adjusting to 10ml with MACS buffer, the number of cells were counted (7×10⁶ ~ 8×10⁶ cells were observed per mouse). After centrifugation at 1500 rpm for 5 minutes, the cells were dissolved with bruff medium (2×10⁵ cells per 100 µl). 100 µl of cells, 20 µl of protein, and 80 µl of cytokine were sequentially placed in a 96-well plate, and 200 µl of PBS was added to all surrounding wells (to prevent evaporation). After 4 days, the cells were re-stimulated to secrete cytokines except 100 µl set aside for ELISA. Cell stimulation cocktail (500x) was diluted in the medium at 1/500 based on a total amount of 200 µl, and 100 µl was added. (-) was applied only to the medium, and pipetting was performed once each. After 4 hours, it was transferred to a cell round well, downed at 2500 rpm for 2 minutes, washed with PBS, and downed again. After pipetting 0.2 µl of CD4-APC per well in 50 µl of PBS, the mixture was kept in the refrigerator for 30 minutes (foil), and then 150 µl of PBS was added and downed. 80 µl of 1X IC fixation buffer was added and it was stored in the refrigerator for 30 minutes. 10X of membrane permeabilization buffer was added to 1X 120 µl and immediately centrifuged. 50 µl of membrane permeabilization buffer and 0.2 µl of IL17A was pipetted for 30 minutes and treated to 21 wells. After washing with the membrane permeabilization solution, it was downed, washed and downed again, then dissolved with PBS, and flow cytometry (FACS) was conducted. CD4 APC (FL-4) for CD4+ cells, IL-17A PE (Phycoerythrin) (FL-2) for Th17 cells, IL-4 PE (FL-2) for Th2 cells, IFN-r FITC (FL-1) for Th1 cells, and Foxp3 FITC (FL-1) for Treg cells was used.

### [1-2] Antibody staining experimental protocol

Each differentiated cell (200 µl) in [1-1] was pipetted and transferred to a round well. For staining of cell surface proteins after centrifugation at 2500 rpm for 2 minutes, the medium was removed and 50 µl of PBS and antibody were mixed per well and pipetted. The plate was wrapped in foil and incubated at 4°C for 30 minutes. Then, 150 µl of PBS was added to each well and centrifuged at 2500 rpm for 2 minutes. For intracellular protein staining, the medium was removed and the fixation/permeation concentration (4x) was diluted to 1x using the fixation/permeation diluent, 80 µl was dissolved per well, the plate was wrapped in foil, and incubated at 4°C for 30 minutes. Then, the 10x permeation buffer was diluted to a 1x permeation buffer using DW, and 120 µl was added per well and centrifuged at 2500 rpm for 2 minutes. Then, 50 µl of 1x permeation buffer and antibody were mixed and pipetted per well. The plate was wrapped in foil and incubated for 30 minutes at 4 °C. 150 µl of 1x permeation buffer was added and centrifuged at 2500 rpm for 2 minutes. 200 µl of PBS was added and dissolved, then washed by centrifugation at 2500 rpm for 2 minutes. In addition, 200 µl of PBS was added and dissolved, followed by FACS analysis.

### [1-3] Results

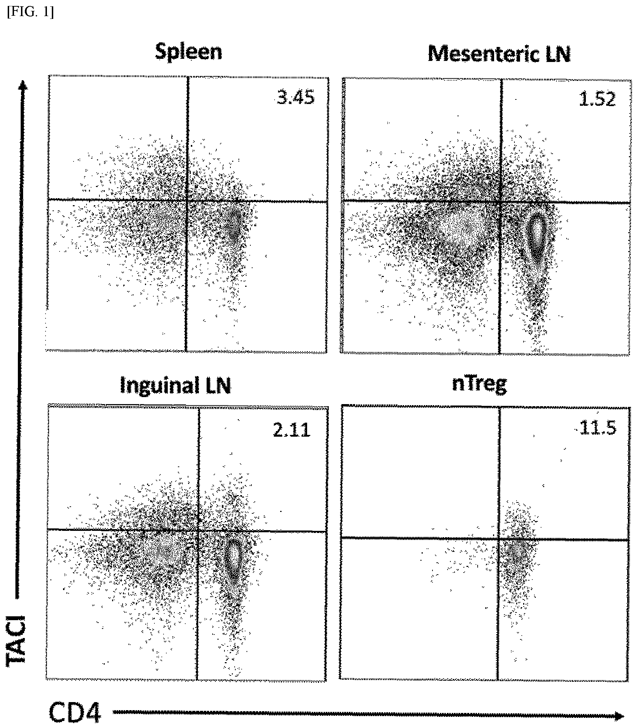

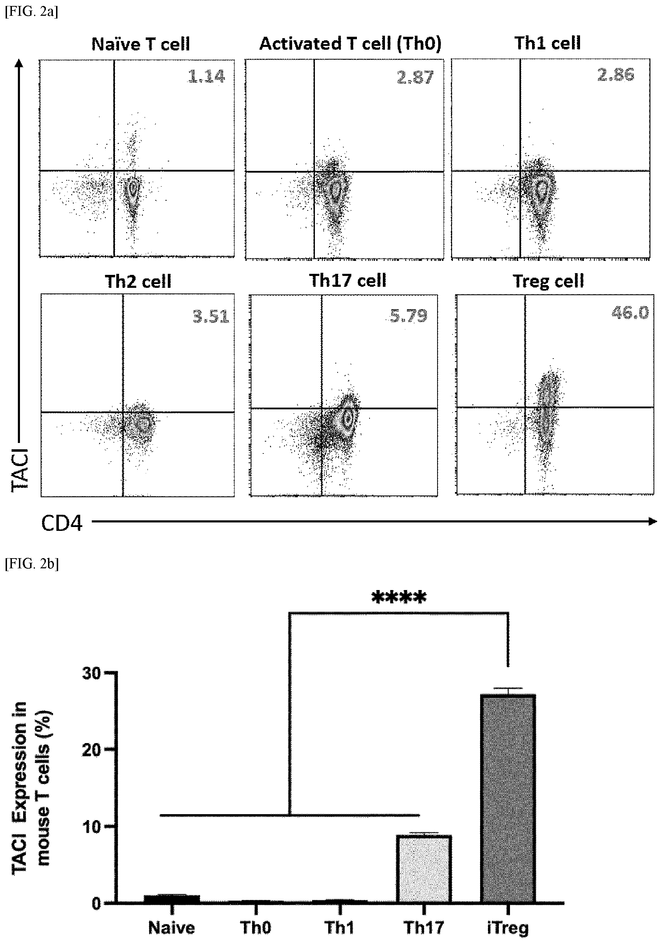

As a result, as illustrated in FIGS. 1 and 2, TACI protein expression was specifically increased in Treg cells.

### [Example 2] Confirmation of TACI expression level by TGF-β concentration

### [2-1] Differentiation protocol for each T-cell subset

After separating the spleen from mice, it was placed in a 60Ψ dish containing 3ml of PBS, rinsed, and then transferred to another 60Ψ dish with 3ml of PBS. After grinding the spleen with the lid of the e-tube, the remaining solution except the tissue was transferred to a 50 ml conical tube, and the lid of the e-tube was washed with PBS and also put in the tube. After centrifugation at 1500 rpm for 5 minutes, the remaining PBS except about 1 ml was aspirated, and the pellet was dissolved with RBC hemolysis buffer (5ml per mouse). The dissolved cells were filtered on a cell strainer in a new 50 ml conical tube, washed with PBS and adjusted to a total volume of 50 ml, and then the number of cells was counted. (Approximately 107 to 108 cells were observed per mouse.) After centrifugation at 1500 rpm for 5 minutes, 400 µl (~30%) of MACS buffer and 100 µl (~30%) of CD4+ cocktail was added per 108 cells and placed in the refrigerator for 10 minutes (tapping for 15 seconds every 1 minute). 300 µl of MACS buffer (~30%) and 200 µl of anti-biotin (~30%) were added to the conical above and placed in the refrigerator for 15 minutes (tapping for 15 seconds every 1 minute). After adjusting to 20 ml with MACS buffer, centrifugation was performed at 1200 rpm for 10 minutes (simultaneously, the LD column was filled with 3 ml of MACS buffer). After aspirating except only 1 ml, it was dissolved with 3 ml of MACS buffer, and after placing a paper cup, ice, and a 50 ml conical under the LD column, it was poured onto the cell filter (repeated twice more). After adjusting to 20 ml with MACS buffer, centrifugation was performed at 1200 rpm for 10 minutes. After aspirating except only 1 ml, 800 µl (~30%) of MACS buffer and 200 µl (~30%) of anti-CD62L+ were added per 108 cells and placed in the refrigerator for 15 minutes (tapping for 15 seconds every 1 minute). After adjusting to 20 ml with MACS buffer, centrifugation was performed at 1200 rpm for 10 minutes (fill LS column with 3 ml of MACS buffer when 5 minutes are left). After aspirating except only 1 ml, it was dissolved with 3 ml of MACS buffer, and after placing a paper cup under the LS column, it was poured onto the cell filter (repeated twice more). The LS column was placed on a 15 ml conical tube and 3 ml of MACS buffer was added, and then the beads were pushed away from the LS column using a cylinder. After adjusting to 10ml with MACS buffer, the number of cells were counted (7×10⁶ ~ 8×10⁶ cells were observed per mouse). After centrifugation at 1500 rpm for 5 minutes, the cells were dissolved with bruff medium (2×10⁵ cells per 100 µl). 100 µl of cells, 20 µl of protein, and 80 µl of cytokine were sequentially placed in a 96-well plate, and 200 µl of PBS was added to all surrounding wells (to prevent evaporation). After 4 days, the cells were re-stimulated to secrete cytokines except 100 µl set aside for ELISA. Cell stimulation cocktail (500x) was diluted in the medium at 1/500 based on a total amount of 200 µl, and 100 µl was added. (-) was applied only to the medium, and pipetting was performed once each. After 4 hours, it was transferred to a cell round well, downed at 2500 rpm for 2 minutes, washed with PBS, and downed again. After pipetting 0.2 µl of CD4-APC per well in 50 µl of PBS, the mixture was kept in the refrigerator for 30 minutes (foil), and then 150 µl of PBS was added and downed. 80 µl of 1X IC fixation buffer was added and it was stored in the refrigerator for 30 minutes. 10X of membrane permeabilization buffer was added to 1X 120 µl and immediately centrifuged. 50 µl of membrane permeabilization buffer and 0.2 µl of IL17A was pipetted for 30 minutes and treated to 21 wells. After washing with the membrane permeabilization solution, it was downed, washed and downed again, then dissolved with PBS, and flow cytometry (FACS) was conducted. CD4 APC (FL-4) for CD4+ cells, IL-17A PE (Phycoerythrin) (FL-2) for Th17 cells, IL-4 PE (FL-2) for Th2 cells, IFN-r FITC (FL-1) for Th1 cells, and Foxp3 FITC (FL-1) for Treg cells was used.

### [2-2] Antibody staining experimental protocol

Each differentiated cell (200 µl) in [2-1] was pipetted and transferred to a round well. For staining of cell surface proteins after centrifugation at 2500 rpm for 2 minutes, the medium was removed and 50 µl of PBS and antibody were mixed per well and pipetted. The plate was wrapped in foil and incubated at 4°C for 30 minutes. Then, 150 µl of PBS was added to each well and centrifuged at 2500 rpm for 2 minutes. For intracellular protein staining, the medium was removed and the fixation/permeation concentration (4x) was diluted to 1x using the fixation/permeation diluent, 80 µl was dissolved per well, the plate was wrapped in foil, and incubated at 4°C for 30 minutes. Then, the 10x permeation buffer was diluted to a 1x permeation buffer using DW, and 120 µl was added per well and centrifuged at 2500 rpm for 2 minutes. Then, 50 µl of 1x permeation buffer and antibody were mixed and pipetted per well. The plate was wrapped in foil and incubated for 30 minutes at 4 °C. 150 µl of 1x permeation buffer was added and centrifuged at 2500 rpm for 2 minutes. 200 µl of PBS was added and dissolved, then washed by centrifugation at 2500 rpm for 2 minutes. In addition, 200 µl of PBS was added and dissolved, followed by FACS analysis.

### [2-3] Results

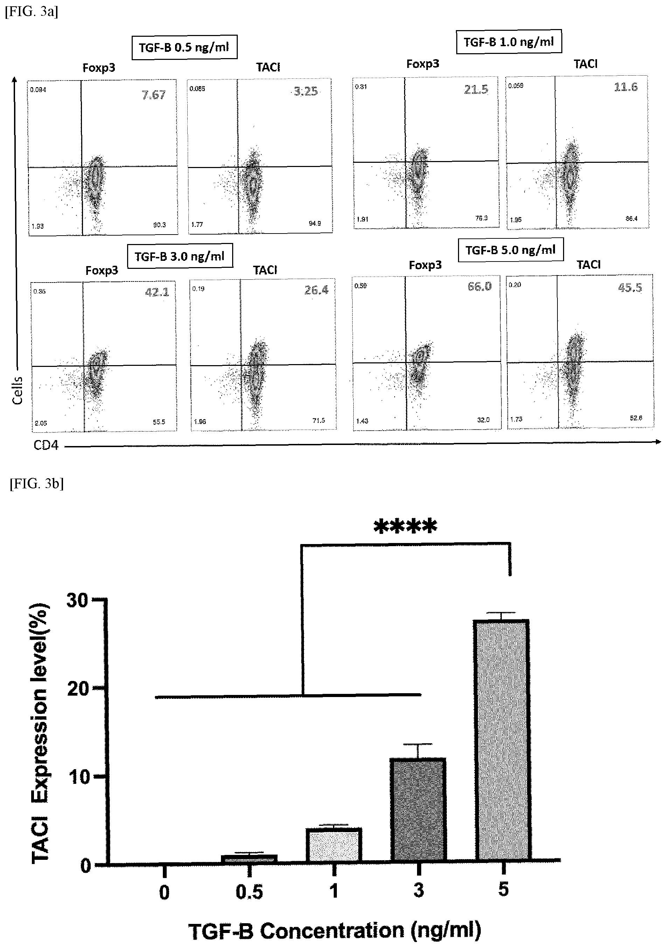

As a result, as illustrated in FIGS. 3a to 3b, TACI expression on the surface of Treg cells is induced by the negative immune modulator TGF-β, as it can be seen that the expression of TACI increases as the concentration of TGF-β increases.

### [Example 3] Confirmation of relationship between Foxp3 expression and TACI expression level

### [3-1] Differentiation protocol of iTreg cells

Here, iTreg cells refer to cells artificially induced to differentiate in a medium containing the following composition, unlikely naturally isolated nTreg cells. For iTreg cells, naive T cells are first isolated from the spleen of mice, and then differentiation into iTreg cells was induced by culturing for 72 hours in a 37°C 5% CO₂ incubator after further including each of the components of Table 1 below in RPMI1640 (Invitrogen Gibco, Grand Island, NY) nutrient medium containing 10% fetal bovine serum (FBS; hyclone, logan, UT).

**[Table 1]**

| Differentiated cell | Composition |
|---|---|
| iTreg | IL-2, TGFβ |

### [3-2] Antibody staining experimental protocol

The expression level of each differentiated cell (200 ul) in [3-1] was confirmed by staining CD69, the activation marker on day 1 after differentiation. In other words, each differentiated cell (200 µl) in [3-1] was pipetted and transferred to a round well. For staining of cell surface proteins after centrifugation at 2500 rpm for 2 minutes, the medium was removed and 50 µl of PBS and antibody were mixed per well and pipetted. The plate was wrapped in foil and incubated at 4°C for 30 minutes. Then, 150 µl of PBS was added to each well and centrifuged at 2500 rpm for 2 minutes. For intracellular protein staining, the medium was removed and the fixation/permeation concentration (4x) was diluted to 1x using the fixation/permeation diluent, 80 µl was dissolved per well, the plate was wrapped in foil, and incubated at 4°C for 30 minutes. Then, the 10x permeation buffer was diluted to a 1x permeation buffer using DW, and 120 µl was added per well and centrifuged at 2500 rpm for 2 minutes. Then, 50 µl of 1x permeation buffer and antibody were mixed and pipetted per well. The plate was wrapped in foil and incubated for 30 minutes at 4°C. 150 µl of 1x permeation buffer was added and centrifuged at 2500 rpm for 2 minutes. 200 µl of PBS was added and dissolved, then washed by centrifugation at 2500 rpm for 2 minutes. In addition, 200 µl of PBS was added and dissolved, followed by FACS analysis.

### [3-3] Results

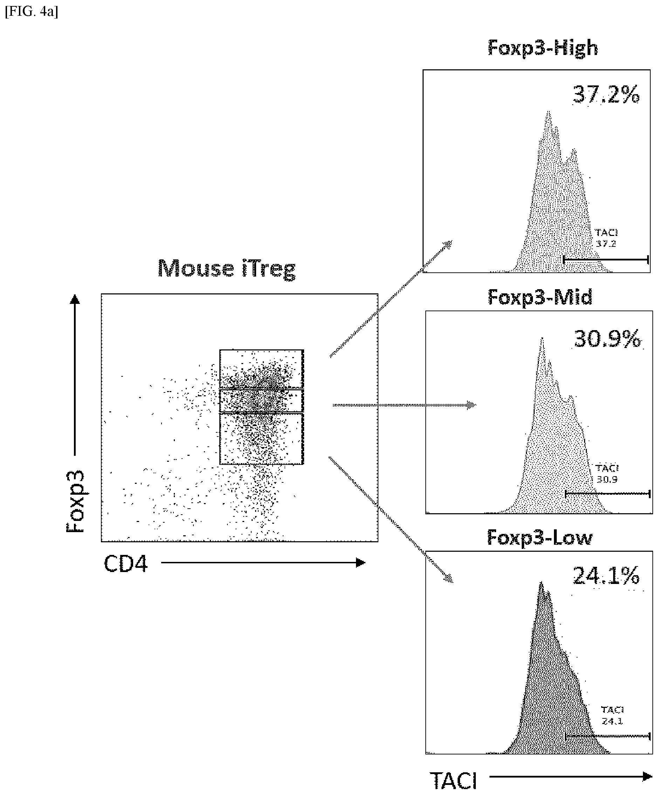

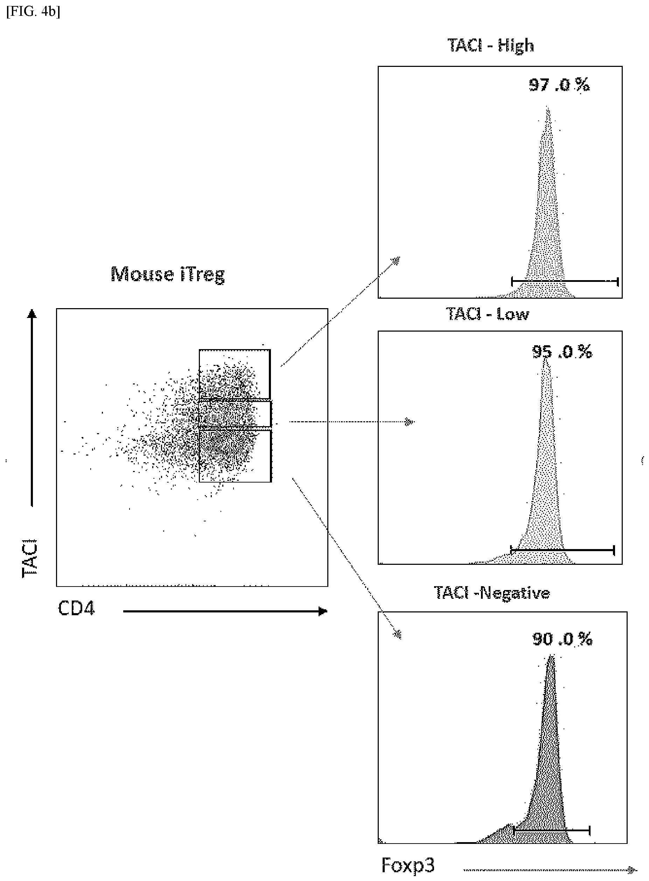

As a result, as illustrated in FIGS. 4a to 4b, TACI expression is correlated with Foxp3 expression, as it can be seen that TACI expression is increased in iTreg cells of mice with high Foxp3 expression.

### [Example 4] Confirmation of whether C-9 antibody inhibits activity during iTreg differentiation

### [4-1] Differentiation protocol of iTreg cells

Here, iTreg cells refer to cells artificially induced to differentiate in a medium containing the following composition, unlikely naturally isolated nTreg cells. For iTreg cells, naive T cells are first isolated from the spleen of mice, and then differentiation into iTreg cells was induced by culturing for 72 hours in a 37°C 5% CO2 incubator after further including each of the components of Table 1 below in RPMI1640 (Invitrogen Gibco, Grand Island, NY) nutrient medium containing 10% fetal bovine serum (FBS; hyclone, logan, UT).

**[Table 2]**

| Differentiated cell | Compsitioin |
|---|---|
| iTreg | IL-2, TGFβ |

### [4-2] Antibody staining protocol

The expression level of each differentiated cell (200 ul) in [4-1] was confirmed by staining CD69, the activation marker on day 1 after differentiation. In other words, each differentiated cell (200 µl) in [4-1] was pipetted and transferred to a round well. For staining of cell surface proteins after centrifugation at 2500 rpm for 2 minutes, the medium was removed and 50 µl of PBS and antibody were mixed per well and pipetted. The plate was wrapped in foil and incubated at 4°C for 30 minutes. Then, 150 µl of PBS was added to each well and centrifuged at 2500 rpm for 2 minutes. For intracellular protein staining, the medium was removed and the fixation/permeation concentration (4x) was diluted to 1x using the fixation/permeation diluent, 80 µl was dissolved per well, the plate was wrapped in foil, and incubated at 4°C for 30 minutes. Then, the 10x permeation buffer was diluted to a 1x permeation buffer using DW, and 120 µl was added per well and centrifuged at 2500 rpm for 2 minutes. Then, 50 µl of 1x permeation buffer and antibody were mixed and pipetted per well. The plate was wrapped in foil and incubated for 30 minutes at 4 °C. 150 µl of 1x permeation buffer was added and centrifuged at 2500 rpm for 2 minutes. 200 µl of PBS was added and dissolved, then washed by centrifugation at 2500 rpm for 2 minutes. In addition, 200 µl of PBS was added and dissolved, followed by FACS analysis.

### [4-3] Results

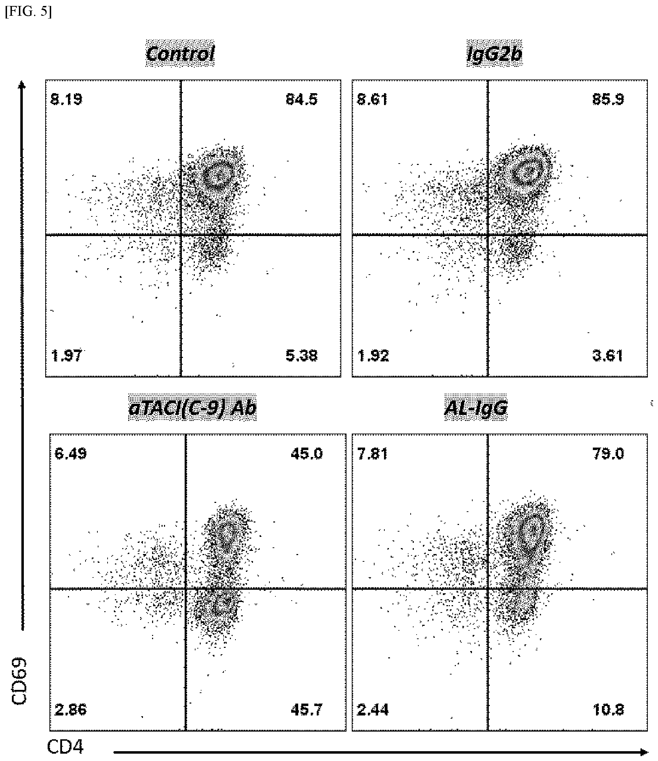

As a result, as illustrated in FIG. 5, the C-9 antibody suppresses differentiation into iTreg cells by suppressing its activity during the process of iTreg differentiation, as it can be seen that the expression of CD69 protein was low when the C-9 (see mouse monoclonal Ab raised against amino acids 42-293 mapping at the C-terminus of TACI of human origin, Santa Cruz [Cat No. sc-365253]) antibody was administered

### [Example 5] Confirmation of whether C-9 antibody inhibits expression of transcription factors

### [5-1] C-9 treatment method during process of CD4 T cell differentiation

After inserting CD4+CD62L+Naive CD4 T cells isolated from mice into a 96 well coated with 0.2ug of anti-CD3 antibody and 1ug of C-9 antibody, the cells were differentiated for about 3 days after treatment with a cytokine mixture required for differentiation into each CD4 T cell subset and 0.2ug of anti-CD28 antibody in a soluble form. Then, TH17 cells were mixed with 1ng/ml TGF-B and 30ng/ml IL-6, and Treg cells were mixed with 1ng/ml TGF-B and 20ng/ml IL-2.

### [5-2] Results

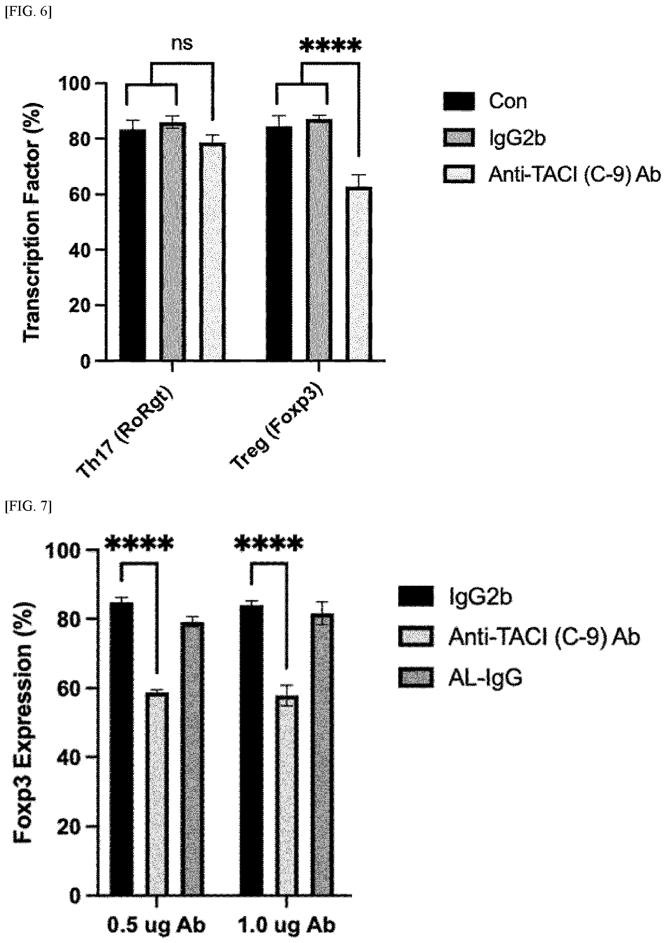

As a result, as illustrated in FIG. 6, the expression of Foxp3, a marker transcription factor important for Treg cells and their function, was reduced when the C-9 antibody was administered. On the other hand, the expression of RORgt, a marker transcription factor in Th17 cells, was not affected. Accordingly, the C-9 antibody selectively inhibits the function of iTreg cells by selectively suppressing activity during the process of iTreg differentiation without affecting other T cell subsets.

### [Example 6] Confirmation of whether C-9 antibody inhibits expression of Foxp3

### [6-1] Confirmation of Foxp3 expression level after treatment with C-9 antibody

After coating the CD4+CD62L+Naive CD4 T cells separated from the mouse with 0.2ug CD3 antibody and C-9 antibodies or AL-IgG on the wells of the 96-well plate according to the indicated concentrations, the expression level of Foxp3 was measured after culturing for 3 days according to the Treg differentiation conditions described in [Example 4] above.

### [6-2] Results

As a result, as illustrated in FIG. 7, the C-9 antibody suppresses differentiation into iTreg cells by suppressing its activity during the process of iTreg differentiation, as it can be seen that the expression of Foxp3 protein in Treg cells is low when the C-9 antibody as administered.

Although the present invention has been described in detail above, it will be obvious to those skilled in the art that these specific techniques are only preferred embodiments, and the scope of the present invention is not limited thereto. Accordingly, the substantial scope of the present invention will be defined by the appended claims and equivalents thereof.

## Claims

1. A pharmaceutical composition for preventing or treating neurodegenerative diseases or neuroinflammatory diseases comprising an antibody or antigen-binding fragment specifically binds to TACI (transmembrane activator and CAML interactor) protein or fragments thereof.

2. The pharmaceutical composition according to claim 1,
wherein the antibody or antigen-binding fragment is human or mouse-derived antibody.

3. The pharmaceutical composition according to claim 1,
wherein the TACI protein or fragments thereof are present in regulatory T cells.

4. The pharmaceutical composition according to claim 1,
wherein the neurodegenerative diseases or neuroinflammatory diseases are at least one selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, cortical degeneration syndrome, multiple system atrophy, progressive supranuclar palsy, nervous system autoimmune disease, spinocerebellar ataxia, inflammatory and neuropathic pain, cerebrovascular disease, spinal cord injury and tauopathy.

5. The pharmaceutical composition according to claim 1,
wherein the pharmaceutical composition is injected into the body through a route selected from oral, intravenous, intramuscular, intraarterial, intramedullary, intradural, intracardiac, transdermal, subcutaneous, intraperitoneal, intranasal, intestinal, topical, sublingual, or rectal.

6. A pharmaceutical composition for prevention or treatment of neurodegenerative diseases or neuroinflammatory diseases comprising an antibody-drug conjugate (ADC) comprising an antibody or antigen-binding fragment specifically binds to TACI (transmembrane activator and CAML interactor) protein or fragments thereof, and drugs.

7. The pharmaceutical composition according to claim 6,
wherein the antibody or antigen-binding fragment specifically binds to TACI protein or fragments thereof are human or mouse-derived antibodies.

8. The pharmaceutical composition according to claim 6,
wherein the TACI protein or fragments thereof are present in regulatory T cells.

9. The pharmaceutical composition according to claim 6,
wherein the neurodegenerative diseases or neuroinflammatory diseases are at least one selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, cortical degeneration syndrome, multiple system atrophy, progressive supranuclar palsy, nervous system autoimmune disease, spinocerebellar ataxia, inflammatory and neuropathic pain, cerebrovascular disease, spinal cord injury and tauopathy.

10. The pharmaceutical composition according to claim 6,
wherein the pharmaceutical composition is injected into the body through a route selected from oral, intravenous, intramuscular, intraarterial, intramedullary, intradural, intracardiac, transdermal, subcutaneous, intraperitoneal, intranasal, intestinal, topical, sublingual, or rectal.

11. A diagnostic composition for neurodegenerative diseases, neuroinflammatory diseases, or cancer comprising an agent for measuring the expression level of TACI (transmembrane activator and CAML interactor) protein, fragments thereof, or a gene encoding the same.

12. The diagnostic composition according to claim 11,
wherein the agent comprises at least one selected from the group consisting of antibodies, oligopeptides, ligands, PNAs (peptide nucleic acids) and aptamers that specifically bind to the protein or fragments thereof.

13. The diagnostic composition according to claim 11,
wherein the agent comprises at least one selected from the group consisting of primers, probes and antisense nucleotides that specifically bind to the gene.

14. A diagnostic kit for neurodegenerative diseases, neuroinflammatory diseases, or cancer comprising the diagnostic composition of any one of claims 11 to 13.

15. A method for providing information for diagnosing neurodegenerative diseases, neuroinflammatory diseases, or cancer comprising the step of measuring the expression level of TACI protein or fragments thereof, or a gene encoding the same, in a biological sample isolated from a target individual.

16. The method according to claim 15,
wherein the biological sample comprises whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluids, meningeal fluids, amniotic fluids, glandular fluids, pancreatic fluids, lymph fluids, pleural fluid, nipple aspirate, bronchial aspirate, synovial fluids, joint aspirate, organ secretions, cells, cell extracts or cerebrospinal fluids.

17. The method according to claim 15,
wherein the method of measuring the expression level of TACI protein or fragments thereof is performed by protein chip assay, immunoassay, ligand binding assay, matrix assisted laser desorption/ionization time of flight mass spectrometry (MALDI-TOF), surface enhanced laser desorption/ionization time of flight mass spectrometry (SELDI-TOF), radioimmunoassay, radial immunodiffusion, ouchterlony immunodiffusion, rocket immunoelectrophoresis, immunohistochemical staining, complement fixation assay, two-dimensional electrophoresis, liquid chromatography-mass spectrometry (LC-MS), liquid chromatography-mass spectrometry/mass spectrometry (LC-MS/MS), Western blotting, or enzyme linked immunosorbent assay (ELISA).

18. The method according to claim 15,
wherein the method of measuring the expression level of the gene encoding TACI protein or fragments thereof is performed by reverse transcription polymerase chain reaction (RT-PCR), competitive reverse transcription polymerase chain reaction (competitive RT-PCR), real-time RT-PCR, RNase protection assay (RPA), Northern blotting or DNA chip.

19. The method according to claim 15,
wherein a high probability of developing a neurodegenerative disease, neuroinflammatory disease, or cancer is predicted when the expression level of TACI protein or fragments thereof, or the gene encoding the same, measured in the biological sample, is reduced compared to the normal control group.

20. A method for screening drugs for treating neurodegenerative diseases, neuroinflammatory disease, or cancer comprising:
the step of measuring the expression level of TACI protein or fragments thereof, or the gene encoding the same, in the biological sample;
the step of contacting the biological sample with a test substance; and
the step of measuring the expression level of TACI protein or fragments thereof, or the gene, in a separated biological sample after contact with the test substance.

21. A pharmaceutical composition for preventing or treating cancer comprising an antibody or antigen-binding fragment specifically binds to TACI (transmembrane activator and CAML interactor) protein or fragments thereof.

22. The pharmaceutical composition according to claim 21,
wherein the antibody or antigen-binding fragment specifically binds to TACI protein or fragments thereof are human or mouse-derived antibodies.

23. The pharmaceutical composition according to claim 21,
wherein the TACI protein or fragments thereof are present in regulatory T cells.

24. The pharmaceutical composition according to claim 21,
wherein the cancer is solid cancer.

25. The pharmaceutical composition according to claim 21,
wherein the cancer is gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer.

26. The pharmaceutical composition according to claim 21,
wherein the pharmaceutical composition is injected into the body through a route selected from oral, intravenous, intramuscular, intraarterial, intramedullary, intradural, intracardiac, transdermal, subcutaneous, intraperitoneal, intranasal, intestinal, topical, sublingual, or rectal.

27. A pharmaceutical composition for prevention or treatment of cancer comprising, an antibody-drug conjugate (ADC) comprising antibodies or antigen-binding fragments specifically binds to TACI (transmembrane activator and CAML interactor) protein or fragments thereof, and drugs.

28. The pharmaceutical composition according to claim 27,
wherein the antibody specific to TACI protein or fragments thereof are human or mouse-derived antibodies.

29. The pharmaceutical composition according to claim 27,
wherein the TACI protein or fragments thereof are present in regulatory T cells.

30. The pharmaceutical composition according to claim 27,
wherein the cancer is solid cancer.

31. The pharmaceutical composition according to claim 27,
wherein the cancer is gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer.

32. The pharmaceutical composition according to claim 27,
wherein the pharmaceutical composition is injected into the body through a route selected from oral, intravenous, intramuscular, intraarterial, intramedullary, intradural, intracardiac, transdermal, subcutaneous, intraperitoneal, intranasal, intestinal, topical, sublingual, or rectal.
